# EUROPEAN PATENT APPLICATION

(11) **EP 4 656 198 A2**
(43) Date of publication of application: **03.12.2025**
(21) Application number: 25175194.7
(22) Date of filing: 08.05.2025
(51) Int. Cl.: A61K 36/074, A61P 11/00

(54) **USE OF GMI FROM GANODERMA LUCIDUM SPORES IN THE PREPARATION OF PHARMACEUTICALS AND FOOD PRODUCTS**

(30) Priority: 24.05.2024 TW 113119442
(71) Applicant: Titan Biological & Agricultural Technology Co., Limited, Wanchai, Hong Kong (HK)
(72) Inventor: CHEN, KUO-CHUAN, 403005 TAICHUNG CITY (TW); HUNG, TSAI-JEN, 403005 TAICHUNG CITY (TW)
(74) Representative: Casalonga

(57) **Abstract**

The present invention relates to the use of GMI (Ganoderma immunomodulatory protein) derived from Ganoderma lucidum spores in the preparation of pharmaceuticals and food products. The aforementioned pharmaceuticals and food products are prepared using GMI produced by fermentation of a genetically modified Pichia pastoris strain Ey72, and are intended for the treatment, prevention, or amelioration of pulmonary fibrosis.

## Description

### BACKGROUND OF INVENTION

### 1. Field of the Invention

The present invention relates to GMI derived from Ganoderma lucidum spores. More specifically, it pertains to the use of such GMI in the preparation of pharmaceuticals and food products.

### 2. Description of Related Art

Idiopathic pulmonary fibrosis (IPF) is a progressive inflammatory condition characterized by the gradual fibrosis of lung tissue, leading to irreversible and chronic decline in lung function. Although the etiology of IPF remains unclear, recent outbreaks of COVID-19 have significantly increased the global incidence of IPF.

Current studies on IPF pathogenesis have shown that transforming growth factor-beta 1 (TGF-β1) plays a central role in promoting fibrotic progression in IPF. Clinically approved medications such as pirfenidone (PFD) and nintedanib are available for treating IPF, with PFD known to inhibit TGF-B1 activity. However, both medications are associated with a variety of adverse effects, including photosensitivity, reduced appetite, gastrointestinal discomfort, nausea, vomiting, abdominal pain, skin rashes, diarrhea, elevated liver enzymes, headaches, and hypertension. These side effects negatively impact the quality of life of IPF patients, and improvements in survival rates have been limited.

Consequently, the biomedical field continues to explore alternative therapies for IPF, including the use of endoplasmic reticulum protein TXNDC5, extracts from Phellinus linteus, and human umbilical mesenchymal stem cells (HUMSCs).

In parallel, Ganoderma lucidum spores have been found to contain an immunomodulatory protein known as GMI. GMI can promote cytokine secretion and activation of immune cells, thereby enhancing immune responses and reducing susceptibility to bacterial infections. In February 2022, the Taiwan Food and Drug Administration (TFDA) announced that a Ganoderma globulin concentrate, produced via fermentation using a genetically modified Pichia pastoris strain Ey72, is approved for use as a food ingredient.

Numerous research efforts have investigated the application of GMI in treating various diseases. For example, US11058746B2 discloses the use of Ganoderma GMI in treating oral submucous fibrosis (OSF). While this prior patent discloses the concept of using Ganoderma GMI to treat fibrosis in various organs, the fibrotic mechanisms of different organs are distinct, and the data presented in that patent pertain only to the efficacy of GMI in treating oral submucous fibrosis. Therefore, it does not provide sufficient evidence to support GMI's efficacy in treating fibrosis in other organs.

Through research on IPF and GMI derived from Ganoderma lucidum spores, the inventors have discovered that GMI exhibits therapeutic, preventive, and/or ameliorative effects against TGF-β-induced pulmonary fibrosis. Currently, there is no known technology or treatment that utilizes GMI for the treatment, prevention, and/or amelioration of TGF-β-induced IPF.

### SUMMARY OF THE INVENTION

The primary objective of the present invention is to provide the use of GMI (Ganoderma immunomodulatory protein) derived from Ganoderma lucidum spores in the preparation of pharmaceutical compositions and food products. It has been demonstrated that GMI possesses therapeutic, preventive, and/or ameliorative effects against TGF-β-induced idiopathic pulmonary fibrosis (IPF).

To achieve the aforementioned objective, the present invention provides the use of GMI derived from Ganoderma lucidum spores, produced via fermentation of yeast strains, in the preparation of pharmaceuticals and food products. The resulting pharmaceutical compositions are intended for the treatment, prevention, or amelioration of pulmonary fibrosis caused by non-viral factors.

Furthermore, the present invention provides a method for treating, preventing, or ameliorating pulmonary fibrosis, comprising administering to a subject GMI derived from Ganoderma lucidum spores, produced via fermentation using a genetically modified Pichia pastoris strain Ey72.

### BRIEF DESCRIPTION OF THE DRAWINGS

Hereinafter, a better embodiments of the present invention are cited and described in further detail with the drawings, wherein:
FIG. 1: Statistical analysis of GMI's effect on LL29 cell viability.
FIG. 2 (A) and (B): Microscopic and quantitative images showing GMI inhibition of TGF-β1-induced LL29 cell migration.
FIG. 3 (A), (B), (C) and (D): Western blot analysis and quantitative data illustrating GMI's anti-fibrotic activity in TGF-β1-induced fibrotic LL29 cells.
FIG. 4 (A) and (B): Graphs showing body weight and weight change rate in BLM-induced pulmonary fibrosis mouse models treated with GMI.
FIG. 5: Survival rate changes in BLM-induced pulmonary fibrosis mouse models following GMI treatment.
FIG. 6 (A) and (B): Lung weight and relative lung weight changes in BLM-induced mice following GMI treatment.
FIG. 7 is a photo of the lung color of mice in the normal group (NT) and each control group (GMI, BLM and PFD).

### DETAILED DESCRIPTION OF THE INVENTION

A preferred embodiments of the invention are described below with reference to the accompanying figures, which serve to illustrate and further explain the invention:
As used herein, the term "treatment" or "treating" refers to a method that achieves a beneficial or desired clinical outcome, including but not limited to therapeutic and/or prophylactic effects. Therapeutic effects relate to the amelioration or eradication of a pathological condition being treated.

The terms "prevention" or "preventing" and "amelioration" or "ameliorating" are used interchangeably herein and refer to the reduction of one or more measurable parameters in a cell, tissue, or subject when compared to an untreated counterpart. Such comparisons may also be made by comparing pre- and post-treatment measurements in the same subject, tissue, or cell type.

As used herein, the term "subject" refers to a human or non-human animal.

In one embodiment, the present invention provides the use of GMI derived from Ganoderma lucidum spores in the preparation of a pharmaceutical composition. The GMI is produced via fermentation using a yeast strain, such as the genetically modified Pichia pastoris strain Ey72, and is intended for treating, preventing, or ameliorating pulmonary fibrosis.

In another embodiment, the invention provides the use of GMI derived from Ganoderma lucidum spores in the preparation of a food product. The GMI is similarly produced using genetically modified Pichia pastoris strain Ey72 and is intended for preventing or ameliorating pulmonary fibrosis.

In some embodiments, the GMI inhibits the progression of pulmonary fibrosis by suppressing the activity of LL29 cells (human lung fibroblasts).

In certain embodiments, GMI suppresses the expression of three fibrosis-associated proteins in LL29 cells: fibronectin, COL1A1 (collagen type I alpha 1), and alpha-smooth muscle actin (α-SMA).

In some embodiments, the pharmaceutical composition comprising GMI, produced using the Pichia pastoris strain Ey72, is specifically indicated for idiopathic pulmonary fibrosis (IPF).

In one embodiment, the concentration of GMI is less than 0.4 µM.

Additionally, the present invention provides a method of treating, preventing, or ameliorating pulmonary fibrosis in a subject, comprising administering GMI derived from Ganoderma lucidum spores, wherein the GMI is produced via fermentation of the Pichia pastoris strain Ey72.

GMI may be administered via non-enteral routes, such as intravenous injection or infusion, intraperitoneal injection, subcutaneous injection, or intramuscular injection. GMI may also be administered orally, including formulations such as tablets, pills, capsules, liquids, gels, syrups, emulsions, suspensions, or other suitable dosage forms comprising carriers and/or excipients.

It will be appreciated by those skilled in the art that various modifications and adaptations of the present invention are possible without departing from the scope of the invention. The following examples are intended solely for illustrative purposes and should not be construed as limiting the scope of the present invention in any way.

### [Examples]

The present invention aims to investigate and verify the anti-fibrotic properties of GMI through in vitro models to counteract TGF-β-induced fibrosis. The therapeutic activity of GMI was assessed using in vitro idiopathic pulmonary fibrosis (IPF) cell models.

### 1. Human In Vitro Cell Assay: Evaluation of GMI on Pulmonary Fibrosis in Human IPF Cell Models

In the in vitro experiments, cells were pre-treated with test compounds and subsequently stimulated with recombinant TGF-β1 to induce fibrotic processes. TGF-B1 plays a critical role in epithelial-mesenchymal transition (EMT), a key driver in the differentiation of fibroblasts into myofibroblasts. LL29 cells, derived from IPF patient lung fibroblasts, were obtained from ATCC^{®}. Upon reaching 70% confluence, cells were pre-treated with selected concentrations of GMI in low-serum media for 2 hours, followed by exposure to 5 ng/mL of recombinant human TGF-β1 (R&D Systems, MN, USA) for 48 hours.

### GMI Source:

GMI used in this study was a concentrated protein extract derived from Ganoderma lucidum spores, manufactured by Mycomagic Biotechnology Co., Ltd. (Taipei, Taiwan). The amino acid sequence of the GMI corresponds to SEQ ID NO: 1.

MSDTALIFTLAWNVKQLAFDYTPNWGRGRPSSFIDTVTFPTVLTDKAYTYRVVVSGKD
LGVRPSYAVESDGSQKINFLEYNSGYGIADTNTIQVYVIDPDTGNNFIVAQWN (SEQ ID
NO:1)

### Cell Acquisition and Culture:

LL29 cells, derived from human IPF lung fibroblasts, were purchased from ATCC^{®}. The cells were cultured in Dulbecco's Modified Eagle Medium (DMEM; Gibco, USA) supplemented with 10% fetal bovine serum (FBS; Gibco, USA) at 37°C in a humidified incubator (95% humidity, 5% CO₂). Cells were subcultured every 2-3 days.

### Cytotoxicity Assay:

Cytotoxicity of GMI was evaluated using LL29 cells and the MTT colorimetric assay (3-(4,5-dimethylthiazol-2-yl)-2,5-diphenyl tetrazolium bromide). LL29 cells were seeded in 96-well plates at a density of 3,000 cells per well. After overnight attachment, cells were treated with various concentrations of GMI for 48 hours. Thereafter, culture medium was replaced with MTT-containing medium (final MTT concentration: 0.5 mg/mL) and incubated at 37°C for 4 hours. A solubilization solution (100 µL) was added and incubated overnight. Absorbance was measured using an ELISA reader at 550-600 nm with a reference wavelength >650 nm.

### Wound Healing Assay:

Cell migration was evaluated using a dual-well culture insert to create a cell-free gap of 500 ± 100 µm. Microscopic images were captured at 0 and 24 hours. The Robust Quantitative Scratch Assay (RQSA) algorithm was used to quantify wound areas. Wound closure was calculated as: Wound healing (%) = ((Areao - Areat) / Areao) × 100%,where Areao is the initial wound area and Areat is the remaining area at time t.

### Western Blot Analysis:

Cell lysates were prepared in RIPA buffer. Equal amounts of protein were subjected to SDS-PAGE and transferred to PVDF membranes. Primary antibodies against fibronectin, COL1A1, and α-SMA were purchased from GeneTex (Taiwan). HRP-conjugated secondary antibodies were obtained from Jackson ImmunoResearch (USA). Detection was performed using the WesternBright^{®} ECL system (Advansta Inc., USA). Protein expression was quantified using Fiji software.

### Statistical Analysis:

Data (N = 3) were analyzed using GraphPad Prism 7.03 (GraphPad Software, USA). Results are presented as mean ± SEM. Statistical significance between two groups was assessed using Student's t-test, while one-way ANOVA followed by Dunnett's multiple comparison test was used for comparisons between control and treatment groups. Statistical significance is indicated by asterisks or hashtags (e.g., * p < 0.05; ** p < 0.01; *** p < 0.001)

### Effect of GMI on LL29 Cell Viability:

To investigate the effect of GMI on the progression of pulmonary fibrosis, the present invention utilized the LL29 cell line, which consists of lung fibroblasts isolated from a patient with idiopathic pulmonary fibrosis (IPF). As a preliminary step, the cytotoxicity of GMI in LL29 cells was assessed using the MTT assay.

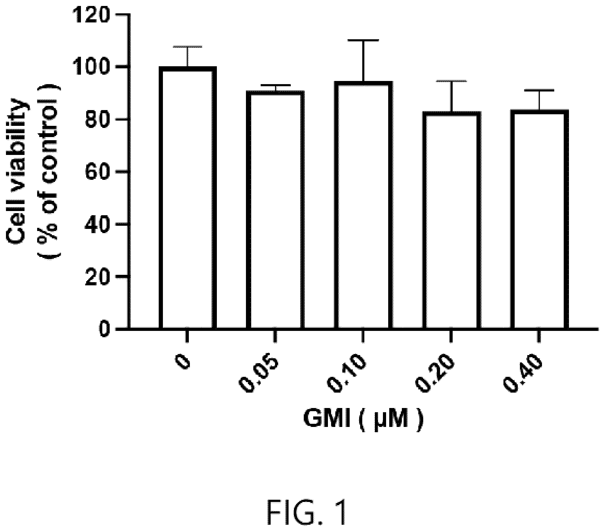

LL29 cells were treated with GMI at concentrations ranging from 0 to 0.4 µM for 48 hours. No significant cytotoxicity was observed at GMI concentrations below 0.4 µM. As shown in FIG. 1, cell viability was determined by MTT assay following treatment with varying concentrations of GMI.

The data are presented as mean ± standard error of the mean (SEM). Statistical analysis was performed using one-way analysis of variance (ANOVA) followed by Dunnett's post hoc multiple comparison test to determine statistical significance between the control group and each treatment group (*p < 0.05; **p < 0.01; ***p < 0.001).

### GMI Inhibits TGF-β1-Induced Migration of LL29 Cells:

Transforming growth factor beta 1 (TGF-β1) at a concentration of 5 ng/mL was used to induce the transdifferentiation of fibroblasts into myofibroblasts. In this study, LL29 cells were co-treated with TGF-β1 (5 ng/mL) and GMI at concentrations of 0.1 µM and 0.2 µM. A positive control group treated with the clinically approved antifibrotic drug pirfenidone (PFD) was included for comparative analysis.

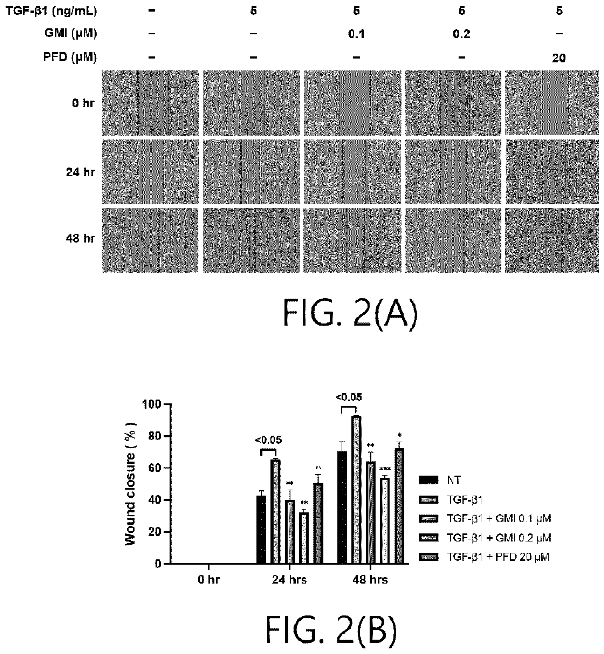

A wound healing assay was performed over a 48-hour period, with observations recorded at 0, 24, and 48 hours after treatment. As shown in FIGs. 2A and 2B, TGF-β1 significantly enhanced wound closure compared to the untreated control (NT group), indicating increased cell migration. In contrast, co-treatment with TGF-β1 and GMI resulted in a dose-dependent suppression of wound closure. Notably, the inhibitory effect of GMI on wound closure was more pronounced than that observed in the PFD-treated group.

FIG. 2A presents representative microscopic images captured at 40× magnification, where the leading edge of migrating cells is marked with dashed lines. FIG. 2B quantifies the wound closure percentage based on the area enclosed between the dashed lines at each time point, with comparisons made relative to the 0-hour control for each group. All data are presented as mean ± standard error of the mean (SEM). Statistical significance was determined using one-way analysis of variance (ANOVA), followed by Dunnett's post hoc multiple comparison test (*p < 0.05; **p < 0.01; ***p < 0.001).

Further analysis of FIGs. 2A and 2B demonstrates that TGF-β1 stimulation enhanced the migratory behavior of LL29 cells, leading to accelerated closure of the wound area between the dashed lines-an indicator of fibrotic progression. Conversely, treatment with GMI reduced the rate of wound closure, suggesting effective inhibition of cell migration and tissue remodeling. A concentration of 0.1 µM GMI was sufficient to achieve measurable inhibition, while 0.2 µM exhibited even greater efficacy. These results confirm that GMI, at both 0.1 µM and 0.2 µM, significantly attenuates LL29 cell migration and the associated fibrotic response.

### GMI Ameliorates the Pathological Mechanisms of TGF-β1-Induced Fibrotic LL29 Cells:

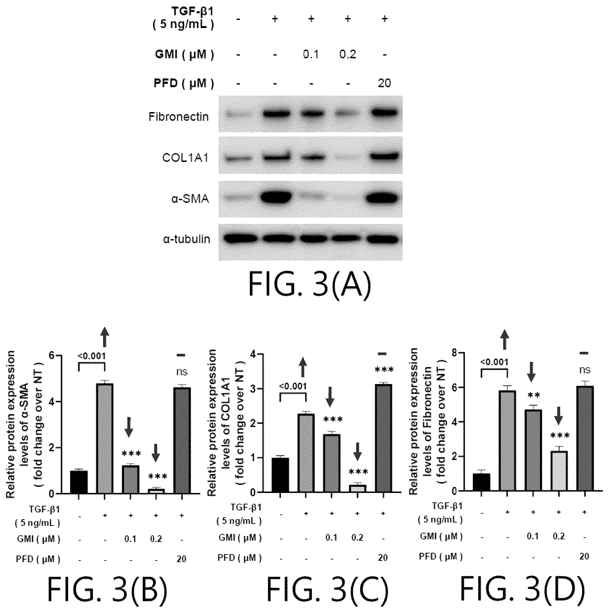

The present invention evaluated the expression levels of fibronectin, COL1A1, and alpha-smooth muscle actin (α-SMA), as these proteins are key markers associated with myofibroblasts and extracellular matrix (ECM) remodeling. As shown in FIG. 3, stimulation with TGF-β1 led to significant upregulation of COL1A1, α-SMA, and fibronectin compared to the untreated control group. Treatment with GMI resulted in a reduction in the expression levels of all three proteins. These results indicate that GMI effectively attenuates TGF-β1-induced fibrotic transformation in LL29 cells, demonstrating anti-fibrotic activity. In contrast, treatment with pirfenidone (PFD) failed to suppress the TGF-β1-induced upregulation of these fibrosis-related markers.

FIG. 3(A) presents the results of Western blot analysis showing the expression of fibronectin, COL1A1, α-SMA, and the internal loading control α-tubulin. FIG. 3(B), (C) and (D) show the quantification of protein expression based on band intensity, represented as fold changes in the ratios of fibronectin/α-tubulin, COL1A1/α-tubulin, and α-SMA/α-tubulin, normalized to the untreated control (NT) group. All values are presented as mean ± standard error of the mean (SEM). Statistical analysis was performed using one-way analysis of variance (ANOVA) followed by Dunnett's post hoc multiple comparison test to assess significance between the control and treated groups (*p < 0.05; **p < 0.01; ***p < 0.001).

Increased expression of α-SMA and enhanced cell migration are closely associated with both acute and chronic fibrotic conditions. Moreover, it is well established that TGF-β1 promotes fibrosis by driving the differentiation of fibroblasts into myofibroblasts. Therefore, the LL29 cell migration model was utilized in this invention to further evaluate and confirm the efficacy of GMI in mitigating pulmonary fibrosis.

### 2. Mouse Model of Pulmonary Fibrosis:

Five groups of C57BL/6J mice (6-8 weeks old, ≥6 mice per group) were used, as shown in Table 1. Four groups were intratracheally administered bleomycin (BLM; 2.5 U/kg) to induce pulmonary fibrosis, while one group served as a normal control. The treatment groups included: BLM-only, BLM + GMI (1 mg/kg), BLM + GMI (2 mg/kg), and BLM + PFD (200 mg/kg). All surviving animals were sacrificed on day 22.

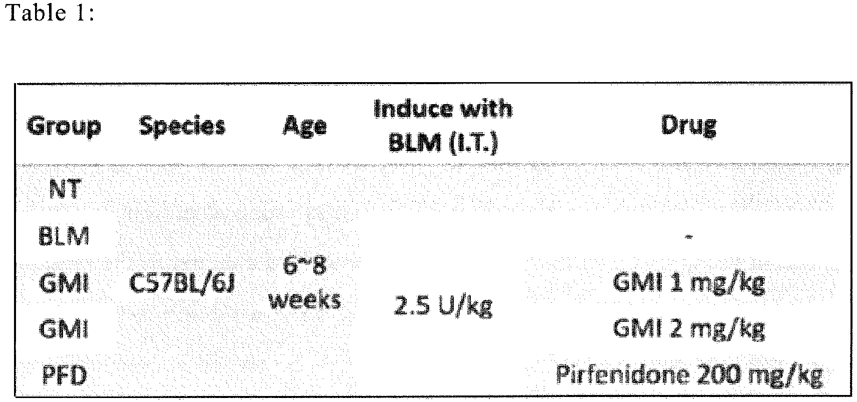

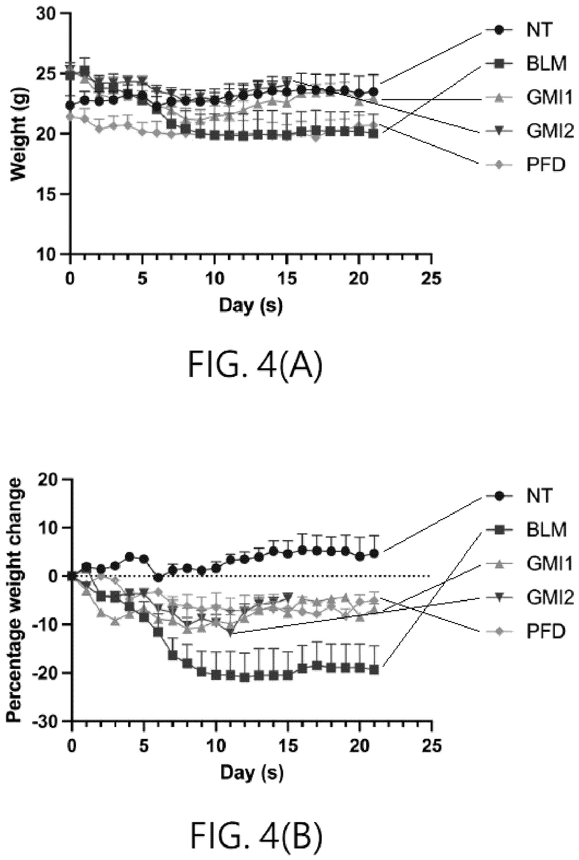

### Body Weight Observation (FIG. 4A, 4B):

Mice treated with BLM showed significant weight loss compared to the normal group. GMI treatment mitigated weight loss effectively.

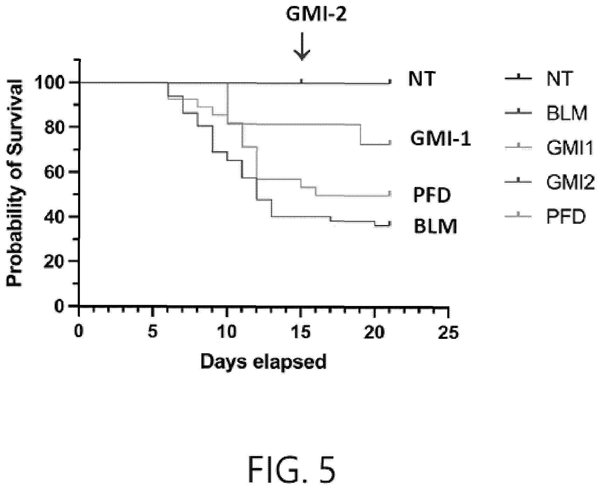

### Survival Rate (FIG. 5):

BLM-only mice showed a survival rate of 40%, while the PFD group had 50% survival. GMI-treated groups showed increased survival: 70% at 1 mg/kg and 100% at 2 mg/kg by day 14, outperforming the clinical standard (PFD).

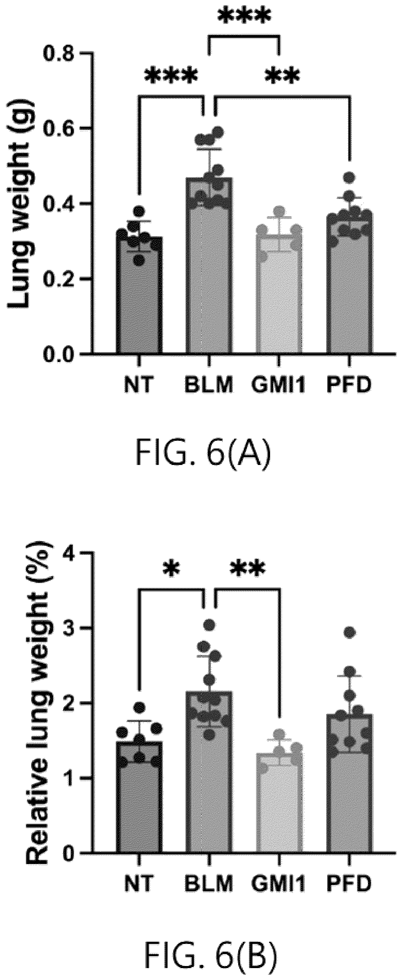

### Lung Weight Analysis (FIG. 6A, 6B):

BLM-induced fibrosis increased lung weight due to inflammation and collagen deposition. GMI-treated mice exhibited normal lung weights and reduced variability in lung weight gain compared to BLM-only mice.

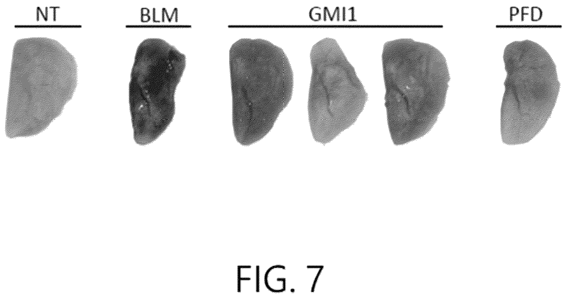

### Lung Appearance (FIG. 7):

Gross examination revealed red (the color shown in FIG. 7 is dark black), inflamed lungs in BLM-treated mice, while GMI- and PFD-treated mice had lungs with color and appearance comparable to the normal group.

The experimental results demonstrate that GMI derived from Ganoderma lucidum spores exhibits significant therapeutic potential in reducing TGF-β1-induced pulmonary fibrosis in both in vitro and in vivo models. GMI was shown to inhibit myofibroblast activation, migration, and fibrotic marker expression more effectively than pirfenidone.

Accordingly, the present invention substantiates the use of GMI for the preparation of pharmaceutical compositions or food products aimed at treating, preventing, or ameliorating pulmonary fibrosis. The disclosed examples are illustrative and not limiting; those skilled in the art may apply variations and modifications without departing from the spirit and scope of the invention, as defined by the claims.

## Claims

1. A use of GMI (Ganoderma immunomodulatory protein) derived from Ganoderma lucidum spores for the preparation of a pharmaceutical composition, wherein the GMI is produced by fermentation of a genetically modified Pichia pastoris strain Ey72, wherein the pharmaceutical composition is for the treatment, prevention, and/or amelioration of pulmonary fibrosis caused by non-viral factors, wherein the pulmonary fibrosis is idiopathic pulmonary fibrosis (IPF), and wherein the GMI comprises an amino acid sequence as set forth in SEQ ID NO: 1.

2. The use of claim 1, wherein the GMI inhibits the activity of LL29 human lung fibroblast cells to suppress the development of pulmonary fibrosis.

3. The use of claim 2, wherein the GMI suppresses the expression of three fibrosis-associated protein markers in LL29 cells, comprising fibronectin, COL1A1, and alpha-smooth muscle actin (α-SMA).

4. The use of claim 1, wherein the concentration of the GMI is less than 0.4 µM.

5. A use of GMI (Ganoderma immunomodulatory protein) derived from Ganoderma lucidum spores for the preparation of a food composition, wherein the GMI is produced by fermentation of a genetically modified Pichia pastoris strain Ey72, wherein the food composition is for the prevention and/or amelioration of pulmonary fibrosis caused by non-viral factors, wherein the pulmonary fibrosis is idiopathic pulmonary fibrosis (IPF), and wherein the GMI comprises an amino acid sequence as set forth in SEQ ID NO: 1.

6. The use of claim 5, wherein the GMI inhibits the activity of LL29 human lung fibroblast cells to suppress the development of pulmonary fibrosis.

7. The use of claim 6, wherein the GMI suppresses the expression of three fibrosis-associated protein markers in LL29 cells, comprising fibronectin, COL1A1, and alpha-smooth muscle actin (α-SMA).

8. The use of claim 5, wherein the concentration of the GMI is less than 0.4 µM.

9. A method for treating, preventing, or ameliorating pulmonary fibrosis in a subject, comprising administering to the subject a GMI produced by fermentation of a genetically modified Pichia pastoris strain Ey72, wherein the pulmonary fibrosis is idiopathic pulmonary fibrosis (IPF), and wherein the GMI comprises an amino acid sequence as set forth in SEQ ID NO: 1.
